# EUROPÄISCHE PATENTSCHRIFT

(11) **EP 4 282 346 B1**
(45) Veröffentlichungstag und Bekanntmachung des Hinweises auf die Patenterteilung: **12.03.2025**
(21) Anmeldenummer: 23174527.4
(22) Anmeldetag: 22.05.2023
(51) Int. Cl.: A61B 17/15

(54) **CHIRURGISCHES INSTRUMENT**
SURGICAL INSTRUMENT
INSTRUMENT CHIRURGICAL

(30) Priorität: 24.05.2022 DE 102022205194
(43) Veröffentlichungstag der Anmeldung: 29.11.2023
(73) Patentinhaber: Aesculap AG, 78532 Tuttlingen (DE)
(72) Erfinder: Firmbach, Franz-Peter, 78576 Emmingen-Liptingen (DE); Anhorn, Svenja, 72535 Heroldstatt (DE)
(74) Vertreter: Patentanwälte Ruff, Wilhelm, Beier, Dauster & Partner mbB

(56) Entgegenhaltungen:
- EP-A2- 1 543 784
- DE-T2- 60 308 475
- DE-T2- 69 324 233
- US-A- 6 056 756
- US-A1- 2017 100 132

## Beschreibung

Die Erfindung betrifft ein chirurgisches Instrumentensystem nach dem Oberbegriff des Anspruchs 1.

Die Verwendung orthopädischer Prothesen als künstlicher Ersatz für beschädigte oder abgenutzte natürliche Knochenstrukturen ist gängige medizinische Praxis. Insbesondere Hüft- und Kniegelenkersatzoperationen gehören mittlerweile zum Standardrepertoire der chirurgischen Orthopädie.

Bei einer Kniegelenkersatzoperation (englisch: Total Knee Arthroplasty (TKA)) werden abgenutzte oder durch Krankheit oder einen Unfall beeinträchtigte Gelenkflächen des Femurs und/oder der Tibia durch eine Kniegelenkprothese ersetzt. Solche Kniegelenkprothesen umfassen üblicherweise eine Femurkomponente, welche am distalen Ende des Femurs implantiert wird, und eine Tibiakomponente, welche am proximalen Ende der Tibia implantiert wird. Um eine einwandfreie Funktion des künstlichen Gelenkersatzes zu gewährleisten, müssen die besagten Komponenten hinsichtlich ihrer Lage und Orientierung in Bezug auf die Anatomie des Patienten und dessen Körperachsen in definierter Weise möglichst präzise positioniert werden. Andernfalls ist mit einem für den Patienten nicht zufriedenstellenden Ergebnis zu rechnen. Hinsichtlich der Positionierung der Komponenten existieren unterschiedliche chirurgische Ansätze.

Ein als Mechanical Alignment bekannter Ansatz sieht vor, dass die Position und Ausrichtung der künstlichen Gelenkachsen der Kniegelenkprothese mechanisch ideal und insoweit ohne Berücksichtigung etwaiger orthopädischer Fehlstellungen des Patienten festgelegt werden. Hierbei dient oftmals die Längsachse der Tibia als Referenzachse für die Ausrichtung und Positionierung. Klinische Studien haben gezeigt, dass der Mechanical Alignment-Ansatz zu einer als unnatürlich empfundenen Funktion des künstlichen Kniegelenks führen kann.

Als ein weiterer Ansatz ist das sogenannte Kinematic Alignment bekannt. Bei dieser Vorgehensweise werden die Femurkomponente und die Tibiakomponente unter Berücksichtigung etwaiger orthopädischer Fehlstellungen des Patienten positioniert. Ziel hierbei ist es, die natürliche und unter Umständen mit Fehlstellungen behaftete Gelenkausrichtung des Patienten wiederherzustellen. Klinische Studien haben gezeigt, dass der Kinematic Alignment-Ansatz oftmals mit einer verbesserten Patientenzufriedenheit einhergeht. Insbesondere die Funktion des künstlichen Kniegelenks wird seitens der Patienten als eher natürlich empfunden.

Mit dem Bestreben, die Patientenzufriedenheit weiter zu verbessern, geht ein grundsätzlicher Bedarf nach möglichst präzisen, einfach verwendbaren und kosteneffizienten chirurgischen Instrumenten zur Umsetzung des Kinematic Alignment einher. Die vorliegende Erfindung befasst sich mit einem solchen chirurgischen Instrument und entsprechenden Instrumentensystemen.

Im Speziellen sieht das Kinematic Alignment vor, dass der distale Femurschnitt in einem Flexions-/Extensions-Winkel von 0° angebracht wird. Der distale Femurschnitt wird hierbei üblicherweise orthogonal zu einer metaphysären anterioren Knochenreferenz ausgerichtet. Der Flexion-/Extensions-Winkel beschreibt in Fachkreisen üblicherweise eine Abweichung gegenüber der orthogonalen Ausrichtung in Bezug auf die besagte knöcherne Referenz. Die Schnittführung erfolgt üblicherweise mittels eines an dem distalen Femur angebrachten Schnittblocks, der auch als Sägeblock oder cutting jig bezeichnet wird. Um den Femurschnitt unter 0°-Flexion/Extension anbringen zu können, ist folglich eine dementsprechende Ausrichtung des Schnittblocks erforderlich. Die Ausrichtung erfolgt üblicherweise mittels eines Referenzblocks oder einer Referenzplatte (englisch: alignment plate), die unter Verwendung eines intramedullären Stabs an den distalen Kondylen des Femurs angelegt wird. Der intramedulläre Stab ist parallel zu der genannten knöchernen Referenz orientiert. Bevor der distale Femurschnitt ausgeführt wird, ist eine Kontrolle der Ausrichtung des Schnittblocks wünschenswert.

Aus der DE 693 24 233 T2 ist ein chirurgisches Instrumentensystem mit den oberbegrifflichen Merkmalen des Anspruch 1 bekannt.

Weitere chirurgische Instrumentensysteme sind aus der EP 1 543 784 A2 und der US 2017/100132 A1 bekannt,

Aufgabe der Erfindung ist es, ein chirurgisches Instrumentensystem bereitzustellen, das eine einfache und präzise Kontrolle der Ausrichtung des Schnittblocks zur distalen Resektion des Femurs ermöglicht und gleichzeitig einen möglichst einfachen Aufbau aufweist.

Diese Aufgabe wird durch das Bereitstellen eines chirurgischen Instrumentensystems mit den Merkmalen des Anspruchs 1 gelöst.

Das erfindungsgemäße chirurgische Instrumentensystem weist ein chirurgisches Instrument und einen Schnittblock zur distalen Resektion eines Femurs auf. Das chirurgische Instrument weist auf: eine Befestigungseinrichtung, die zur direkten lösbaren Befestigung an dem Schnittblock eingerichtet ist, eine Tasteinrichtung mit einer posterior orientierten Referenzebene, die zur Anlage an einer anterioren Fläche des Femurs eingerichtet ist, und eine Lagereinrichtung zur Positionierung der Referenzebene, wobei die Lagereinrichtung wenigstens ein Schwenklager mit einer anteroposterior orientierten Schwenkachse aufweist, um welche die Referenzebene relativ zu der Befestigungseinrichtung schwenkbeweglich verlagerbar ist. Durch die erfindungsgemäße Lösung kann die Referenzebene der Tasteinrichtung relativ zu der Befestigungseinrichtung rotatorisch um die Schwenkachse verlagert werden. In der Verwendung des chirurgischen Instruments erlaubt dies eine Relativpositionierung der Referenzebene in Bezug auf den Schnittblock und damit auch in Bezug auf den Femur. Hierdurch wird eine möglichst präzise Positionierung und folglich eine verbesserte Anlage der Referenzebene an der anterioren Fläche des Femurs ermöglicht. Die verbesserte Anlage erlaubt eine besonders einfache und präzise Kontrolle der Ausrichtung des Schnittblocks. Die Befestigungseinrichtung ist zur direkten, d.h. unmittelbaren, Befestigung an dem Schnittblock eingerichtet. Die Befestigungseinrichtung dient der lösbaren Befestigung des chirurgischen Instruments. Die Befestigung ist als Steckverbindung ausgestaltet. Die Tasteinrichtung weist die Referenzebene auf und dient der eigentlichen Kontrolle der Ausrichtung des Schnittblocks. Zu diesem Zweck ist die Referenzebene zur Anlage an der besagten anterioren Fläche des Femurs, die auch als anteriorer Schild bezeichnet werden kann, eingerichtet. Bei einer Ausgestaltung ist die Referenzebene durch eine Fläche eines Bauteils und/oder eines Abschnitts der Tasteinrichtung gebildet. Bei einer weiteren Ausgestaltung ist die Referenzebene gleichsam durch mehrere Abschnitte der Tasteinrichtung aufgespannt. Die Referenzebene ist posterior orientiert. Mit anderen Worten ausgedrückt ist die Referenzebene planparallel zu einer frontalen Ebene des Patienten. Die Lagereinrichtung dient der Positionierung der Referenzebene in Relation zu der Befestigungseinrichtung. Zu diesem Zweck weist die Lagereinrichtung wenigstens das besagte Schwenklager mit der anteroposterior orientierten Schwenkachse auf. Die Schwenkachse und die Referenzebene sind orthogonal zueinander orientiert. Bei einer Ausgestaltung der Erfindung ist das Schwenklager ein gesondertes Bauteil oder eine gesonderte Baugruppe, welche zum einen mit der Befestigungseinrichtung und zum anderen mit der Tasteinrichtung verbunden ist. Bei einer weiteren Ausgestaltung ist das Schwenklager durch Bauteile und/oder Abschnitte der Befestigungseinrichtung einerseits und der Tasteinrichtung andererseits gebildet.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander und können selbstverständlich in Bezug zu nicht mit der Anatomie des Patienten in Verbindung stehenden X-, Y- und Z-Achsen gesetzt werden. Beispielsweise kann die proximal-distale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnung wegen werden nachfolgend in erster Linie die besagten anatomischen Lage- und Richtungsbezeichnungen verwendet. Weiter werden Bezeichnungen wie "Rückseite" einer Komponente oder eines Abschnitts des chirurgischen Instruments, beispielsweise des Schnittblocks, in Bezug auf eine proximal gerichtete Blickrichtung verwendet. Dementgegen werden Bezeichnungen wie "Vorderseite" in Bezug auf eine distal gerichtete Blickrichtung verwendet.

In Ausgestaltung der Erfindung weist die Lagereinrichtung wenigstens eine erste Linearführung auf, mittels derer die Referenzebene relativ zu der Befestigungseinrichtung translatorisch entlang einer anteroposterior orientierten ersten Lagerachse verlagerbar ist. Hierdurch wird der Relativbeweglichkeit der Referenzebene ein weiterer Freiheitsgrad hinzugefügt, nämlich ein translatorischer Freiheitsgrad entlang der ersten Lagerachse. Die erste Lagerachse ist anteroposterior orientiert und damit parallel zu der Schwenkachse ausgerichtet. Bei einer Ausgestaltung ist die erste Lagerachse koaxial zu der Schwenkachse. Durch die Relativbeweglichkeit entlang der ersten Lagerachse wird eine nochmals präzisere Positionierung der Referenzebene und folglich eine nochmals verbesserte Anlage an der anterioren Fläche des Femurs ermöglicht. Dies erlaubt eine weiter verbesserte Kontrolle der Ausrichtung des Schnittblocks. Bei einer Ausgestaltung der Erfindung ist wenigstens die Referenzebene entlang der ersten Lagerachse verlagerbar. Bei einer weiteren Ausgestaltung ist die gesamte Tasteinrichtung entlang der ersten Lagerachse verlagerbar.

In weiterer Ausgestaltung der Erfindung weist die Lagereinrichtung wenigstens eine zweite Linearführung auf, mittels derer die Referenzebene relativ zu der Befestigungseinrichtung translatorisch entlang einer wenigstens überwiegend proximodistal orientierten zweiten Lagerachse verlagerbar ist. Hierdurch wird der Relativbeweglichkeit der Referenzebene ein weiterer Freiheitsgrad hinzugefügt, nämlich ein translatorischer Freiheitsgrad entlang der zweiten Lagerachse. Die Ausrichtung der zweiten Lagerachse ist abhängig von der jeweiligen Schwenkstellung um die Schwenkachse und wenigstens überwiegend proximodistal orientiert. Hierdurch kann die Referenzebene wenigstens überwiegend in Längsrichtung des Femurs positioniert werden. Dies ermöglicht eine nochmals präzisere Positionierung und folglich eine nochmals verbesserte Anlage an der anterioren Fläche des Femurs. Die zweite Lagerachse ist orthogonal zu der Schwenkachse orientiert. Die zweite Lagerachse ist in einer Ebene längserstreckt, die planparallel zu der Referenzebene orientiert ist.

In weiterer Ausgestaltung der Erfindung weist die Tasteinrichtung ein um die Schwenkachse schwenkbewegliches Strebenelement auf, welches entlang seiner Längsrichtung zwischen einem ersten Ende und einem zweiten Ende längserstreckt ist, wobei die Referenzebene an einer posterior orientierten Unterseite des Strebenelements angeordnet oder ausgebildet ist. Die Lagereinrichtung ist vorzugsweise an dem zweiten Ende des Strebenelements angeordnet. Das Strebenelement ragt wenigstens überwiegend proximal von der Befestigungseinrichtung ab, wobei die Längsrichtung des Strebenelements in Abhängigkeit einer Schwenkstellung um die Schwenkachse unterschiedlich orientiert ist. Sofern das chirurgische Instrument eine erste Linearführung gemäß einer der vorhergehenden Ausgestaltungen aufweist, ist bei einer Ausgestaltung das gesamte Strebenelement translatorisch entlang der ersten Lagerachse verlagerbar. Bei einer weiteren Ausgestaltung ist lediglich die Referenzebene entlang der ersten Lagerachse translatorisch verlagerbar, wobei das Strebenelement im Übrigen in Bezug auf die erste Lagerachse feststeht.

In weiterer Ausgestaltung der Erfindung weist das Schwenklager eine Zapfenaufnahme und ein komplementäres Zapfenelement auf, welches um die Schwenkachse schwenkbeweglich in der Zapfenaufnahme gelagert ist, wobei das Zapfenelement an dem zweiten Ende des Strebenelements und die Zapfenaufnahme an der Befestigungseinrichtung angeordnet ist oder umgekehrt. Das Zapfenelement greift axial entlang der Schwenkachse in die Zapfenaufnahme ein. Das Zapfenelement und die Zapfenaufnahme wirken gleitbeweglich zusammen. Bei dieser Ausgestaltung weist das Schwenklager eine besonders einfache und robuste Konstruktion auf.

In weiterer Ausgestaltung der Erfindung ist das Zapfenelement entlang der Schwenkachse, vorzugsweise begrenzt, linearbeweglich in der Zapfenaufnahme gelagert. Durch die begrenzt linearbewegliche Lagerung des Zapfenelements kann das Strebenelement - und damit die Referenzebene - entlang der Schwenkachse, d.h. anteroposterior, translatorisch verlagert werden. Mit anderen Worten ausgedrückt bildet das Schwenklager bei dieser Ausgestaltung gleichsam eine erste Linearführung gemäß einer der vorhergehenden Ausgestaltungen. Diese Ausgestaltung ermöglicht eine relative Positionierung der Referenzebene in Bezug auf wenigstens zwei Freiheitsgrade (um und entlang der Schwenkachse), wobei gleichzeitig ein besonders einfacher Aufbau des chirurgischen Instruments erreicht oder beibehalten wird.

In weiterer Ausgestaltung der Erfindung weist das Strebenelement einen ausgehend von dem zweiten Ende gerade längserstreckten ersten Strebenabschnitt, dessen posterior orientierte Unterseite die Referenzebene bildet, einen zweiten Strebenabschnitt, welcher ausgehend von dem ersten Ende anterior parallel versetzt zu dem ersten Strebenabschnitt längserstreckt ist, und einen dritten Strebenabschnitt, welcher wenigstens überwiegend anteroposterior längserstreckt ist und den ersten Strebenabschnitt und den zweiten Strebenabschnitt miteinander verbindet, auf. Folglich ist das Strebenelement im weitesten Sinne abgekröpft. Der erste Strebenabschnitt ist posterior von dem zweiten Strebenabschnitt beabstandet. Der zweite Strebenabschnitt ist anterior von dem ersten Strebenabschnitt beabstandet. Der dritte Strebenabschnitt ist in Längsrichtung des Strebenelements zwischen dem ersten Strebenabschnitt und dem zweiten Strebenabschnitt angeordnet.

In weiterer Ausgestaltung der Erfindung weist die Tasteinrichtung ein Schlittenelement und ein Tastelement auf, wobei das Schlittenelement relativ zu dem Strebenelement in Längsrichtung des Strebenelements linearbeweglich an demselben gelagert ist, wobei das Tastelement relativ zu dem Schlittenelement orthogonal zu der Längsrichtung des Strebenelements und/oder anteroposterior linearbeweglich an dem Schlittenelement gelagert ist, und wobei die Referenzebene einends des Tastelements angeordnet oder ausgebildet ist. Diese Ausgestaltung der Erfindung ermöglicht eine besonders präzise und variable Positionierung der Referenzebene in Bezug auf die Befestigungseinrichtung. Das Schlittenelement ist entlang des Strebenelements linearbeweglich an demselben gelagert. Sofern die Lagereinrichtung eine zweite Linearführung gemäß einer der vorhergehenden Ausgestaltungen aufweist, bildet die Längsrichtung des Strebenelements gleichsam deren zweite Lagerachse. Das Tastelement - und damit die an demselben angeordnete oder ausgebildete Referenzebene - ist relativ zu dem Schlittenelement orthogonal zu der Längsrichtung des Strebenelements linearbeweglich. Folglich ist das Tastelement anteroposterior linearbeweglich. Sofern die Lagereinrichtung eine erste Linearführung gemäß einer der vorhergehenden Ausgestaltungen aufweist, bildet die linearbewegliche Lagerung des Tastelements an dem Schlittenelement gleichsam deren erste Lagerachse. Das Schlittenelement ist zwischen dem ersten Ende und dem zweiten Ende des Strebenelements relativ zu demselben verlagerbar. Das Schlittenelement ist vorzugsweise gleitbeweglich an dem Strebenelement geführt. Das Tastelement ist vorzugsweise gleitbeweglich an dem Schlittenelement geführt.

In weiterer Ausgestaltung der Erfindung ist das Tastelement relativ zu dem Schlittenelement um eine anteroposterior orientierte Drehachse rotierbar an dem Schlittenelement gelagert. Hierdurch kann eine nochmals verbesserte Positionierung der einends an dem Tastelement angeordneten oder ausgebildeten Referenzebene erreicht werden. Vorzugsweise ist das Tastelement entlang der Drehachse linearbeweglich an dem Schlittenelement gelagert.

Gemäß der Erfindung ist die Befestigungseinrichtung zur direkten lösbaren Befestigung an dem Schnittblock eingerichtet und weist ein mediolateral und anteroposterior eben erstrecktes Steckelement auf, welches zum Ausbilden einer form- und/oder kraftschlüssigen Steckverbindung mit einem Führungsschlitz des Schnittblocks eingerichtet ist. Das Steckelement ist lösbar in den Führungsschlitz eingesteckt. Der Führungsschlitz des Schnittblocks ist - in einem nicht mit dem Steckelement zusammengesteckten Zustand - zur Aufnahme und Führung eines Sägeblatts eingerichtet. Mit anderen Worten ausgedrückt dient der Führungsschlitz einer Führung des Sägeblatts beim Anbringen des distalen Femurschnitts. Das Steckelement ist zum Einstecken in den besagten Führungsschlitz eingerichtet und komplementär zu diesem gestaltet. Hierdurch wird eine besonders einfache und dennoch zuverlässige lösbare Befestigung des chirurgischen Instruments an dem Schnittblock ermöglicht. Durch die direkte Befestigung an dem Schnittblock kann dessen Ausrichtung besonders präzise und zuverlässig kontrolliert werden.

Der Schnittblock weist den in einer mediolateral und anteroposterior erstreckten Führungsebene angeordneten Führungsschlitz zur Aufnahme und Führung eines Sägeblatts aufweist, wobei die Befestigungseinrichtung des chirurgischen Instruments direkt an dem Schnittblock befestigt ist, und wobei die Referenzebene orthogonal zu der Führungsebene orientiert ist. Der Schnittblock kann auch als Femurschnittblock oder Femursägeblock bezeichnet werden und ist zur Führung des Sägeblatts bei der Anbringung des distalen Femurschnitts vorgesehen. Zu diesem Zweck weist der Schnittblock den besagten Führungsschlitz auf. Dieser ist in der Führungsebene plan erstreckt. Die Führungsebene ist mediolateral und anteroposterior ausgedehnt. Die Befestigungseinrichtung ist direkt an dem Schnittblock befestigt, nämlich mittels des Steckelements. Die Referenzebene ist orthogonal zu der Führungsebene orientiert. Hierdurch kann die für das Kinematic Alignment üblicherweise verwendete Ausrichtung mit einem Flexions-/Extensions-Winkel von 0° besonders einfach und präzise kontrolliert werden.

In weiterer Ausgestaltung der Erfindung ist ein Referenzblock vorhanden, welcher eine proximal orientierte Blockrückseite zur Anlage an distalen Kondylen des Femurs aufweist, wobei der Schnittblock lösbar an dem Referenzblock befestigt ist, und wobei die Führungsebene planparallel und die Referenzebene orthogonal zu der Blockrückseite orientiert sind. Die proximal orientierte Blockrückseite dient einer distalen Referenzierung des Referenzblocks und damit auch des an demselben lösbar befestigten Schnittblocks. Die Führungsebene des Schnittblocks ist planparallel und proximal versetzt zu der Blockrückseite angeordnet. Die Referenzebene ist orthogonal zu der Blockrückseite orientiert.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine Ausführungsform eines erfindungsgemäßen chirurgischen Instruments,
- Fig. 2: in schematischer Perspektivdarstellung eine Ausführungsform eines erfindungsgemäßen chirurgischen Instrumentensystems umfassend das chirurgische Instrument nach Fig. 1 und einen Femurschnittblock,
- Fig. 3: in schematischer Perspektivdarstellung eine intraoperative Situation unter Verwendung des Instrumentensystems nach Fig. 2,
- Fig. 4: das chirurgische Instrumentensystem nach Fig. 2 in einer schematischen Seitenansicht mit medialer Blickrichtung,
- Fig. 5: das chirurgische Instrumentensystem nach den Fig. 2 und 4 in einer schematischen Aufsicht mit posteriorer Blickrichtung,
- Fig. 6: eine weitere schematische Aufsicht gemäß Fig. 5, wobei eine Tasteinrichtung des chirurgischen Instruments schwenkbeweglich verlagert ist,
- Fig. 7: in schematischer Perspektivdarstellung eine weitere Ausführungsform eines erfindungsgemäßen chirurgischen Instruments,
- Fig. 8: das chirurgische Instrument nach Fig. 7 in einer schematischen Seitenansicht mit medialer Blickrichtung,
- Fig. 9, 10: schematische Perspektivdarstellungen einer weiteren Ausführungsform eines erfindungsgemäßen chirurgischen Instrumentensystems mit einem chirurgischen Instrument nach den Fig. 7 und 8 und einem Femurschnittblock und
- Fig. 11: in schematischer Perspektivdarstellung eine intraoperative Situation unter Verwendung des chirurgischen Instrumentensystems nach den Fig. 9 und 10.

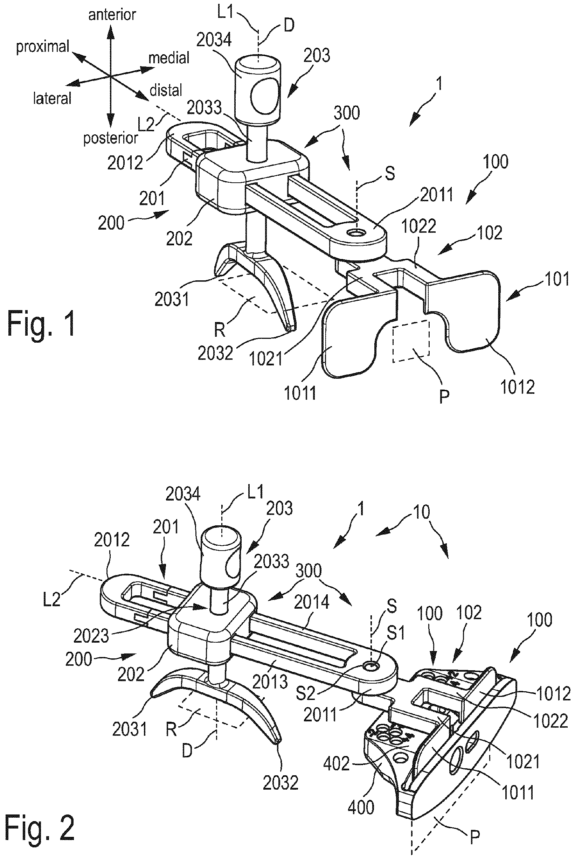

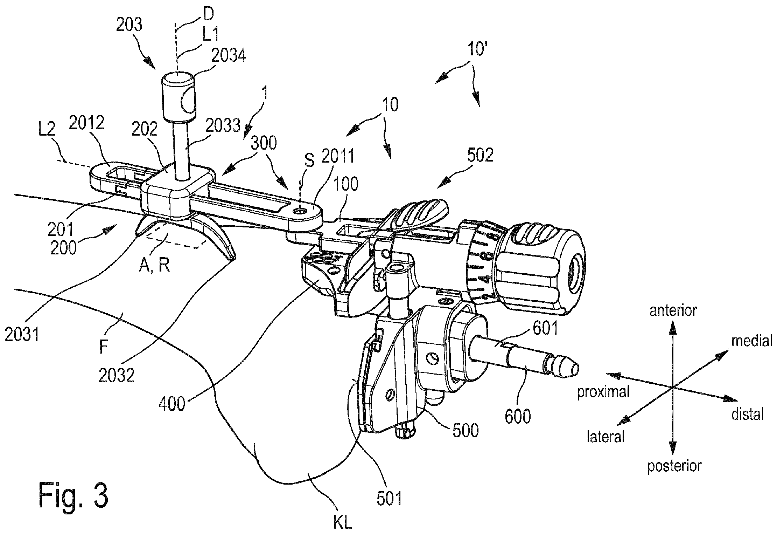

Gemäß Fig. 1 ist ein chirurgisches Instrument 1 zur Verwendung bei einer Kniegelenkersatzoperation vorgesehen und weist eine Befestigungseinrichtung 100, eine Tasteinrichtung 200 und eine Lagereinrichtung 300 auf. Das chirurgische Instrument 1 ist auf noch näher beschriebene Weise zur Kontrolle einer Ausrichtung eines Schnittblocks 400 zur distalen Resektion eines Femurs F eingerichtet (siehe Fig. 3).

Die Befestigungseinrichtung 100 ist zur direkten lösbaren Befestigung des chirurgischen Instruments 1 an dem Schnittblock 400 eingerichtet.

Die Tasteinrichtung 200 weist eine posterior orientierte Referenzebene R auf. Die Referenzebene R ist zur Kontrolle der Ausrichtung des Schnittblocks 400 an einer anterioren Fläche A des Femurs F eingerichtet.

Die Lagereinrichtung 300 dient einer verbesserten Positionierung der Referenzebene R in Bezug auf den Femur F. Zu diesem Zweck weist die Lagereinrichtung 300 wenigstens ein Schwenklager mit einer Schwenkachse S auf. Die Schwenkachse S ist orthogonal zu der Referenzebene R und/oder anteroposterior orientiert. Die Referenzebene R ist relativ zu der Befestigungseinrichtung 100 um die Schwenkachse S schwenkbeweglich. In einer noch näher beschriebenen intraoperativen Situation (siehe Fig. 3) ist die Referenzebene R hierdurch besonders präzise relativ zu der anterioren Fläche A des Femurs F ausrichtbar. Dies erlaubt eine verbesserte Kontrolle der Ausrichtung des Schnittblocks 400.

Das chirurgische Instrument 1 und der Schnittblock 400 bilden vorliegend ein chirurgisches Instrumentensystem 10 (Fig. 2). Das Instrumentensystem 10 ist vorliegend Bestandteil eines (übergeordneten) chirurgischen Instrumentensystems 10', welches neben dem chirurgischen Instrument 1 und dem Schnittblock 400 zudem einen Referenzblock 500 und einen intramedullären Stab 600 umfasst. Auf das Zusammenwirken der unterschiedlichen Komponenten des Instrumentensystems 10' und deren spezifische Funktion wird nachfolgend noch im Detail eingegangen. Zunächst wird der weitere Aufbau des chirurgischen Instruments 1 näher erläutert:
Vorliegend weist die Lagereinrichtung 300 zudem eine erste Linearführung mit einer ersten Lagerachse L1 auf. Die Referenzebene R ist translatorisch entlang der ersten Lagerachse L1 relativ zu der Befestigungseinrichtung 100 verlagerbar. Die erste Lagerachse L1 ist parallel zu der Schwenkachse S und/oder orthogonal zu der Referenzebene R orientiert.

Bei der gezeigten Ausführungsform weist die Lagereinrichtung 300 zudem eine zweite Linearführung mit einer zweiten Lagerachse L2 auf. Die Referenzebene R ist translatorisch entlang der zweiten Lagerachse L2 relativ zu der Befestigungseinrichtung 100 verlagerbar. Die zweite Lagerachse L2 ist orthogonal zu der Schwenkachse S und/oder orthogonal zu der ersten Lagerachse L1 orientiert.

Vorliegend ist die Referenzebene R zudem um die Lagerachse L1 rotatorisch verlagerbar.

Die Lagereinrichtung 300 erlaubt bei der gezeigten Ausführungsform eine Relativbeweglichkeit der Referenzebene R mit einem rotatorischen Freiheitsgrad um die Schwenkachse S, einem translatorischen Freiheitsgrad entlang der Lagerachse L1, einem weiteren translatorischen Freiheitsgrad entlang der zweiten Lagerachse L2 und einem weiteren rotatorischen Freiheitsgrad um die erste Lagerachse L1.

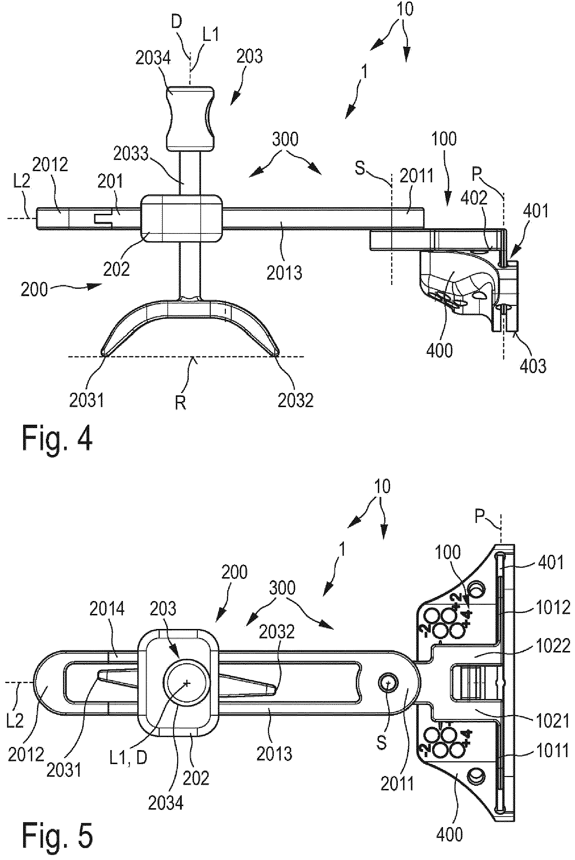

Die Befestigungseinrichtung 100 ist bei der gezeigten Ausführungsform zur direkten lösbaren Befestigung an dem Schnittblock 400 eingerichtet (siehe insbesondere Fig. 2). Der Schnittblock 400 kann auch als Sägeblock oder cutting jig bezeichnet werden. Der Schnittblock 400 ist zur Führung eines Sägeblatts beim Anbringen eines distalen Femurschnitts vorgesehen und weist zu diesem Zweck einen Führungsschlitz 401 auf. Der Führungsschlitz 401 ist zur Aufnahme und Führung des Sägeblatts eingerichtet und reicht von einer Blockoberseite 402 bis auf eine gegenüberliegende Blockunterseite 403 (siehe insbesondere Fig. 4). Der Führungsschlitz 401 definiert eine Führungsebene P, in welcher das besagte Sägeblatt beim Anbringen des distalen Femurschnitts geführt ist.

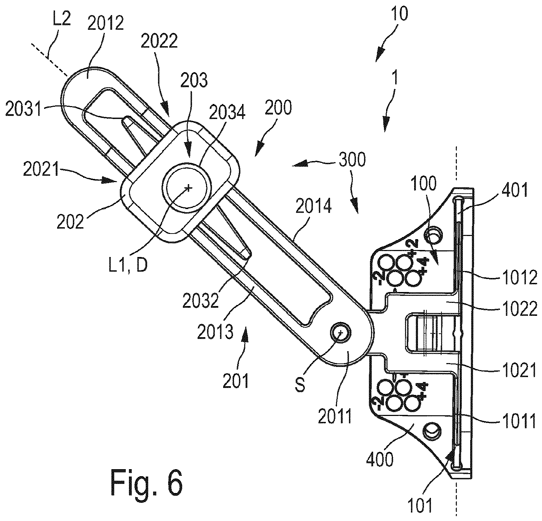

Zur lösbaren Befestigung an dem Schnittblock 400 weist die Befestigungseinrichtung 100 ein Steckelement 101 auf. Das Steckelement 101 ist zum Ausbilden einer form- und/oder kraftschlüssigen Steckverbindung mit dem Führungsschlitz 401 eingerichtet. Das Steckelement 101 ist dementsprechend komplementär zu dem Führungsschlitz 401 gestaltet, dimensioniert und/oder ausgerichtet. Das Steckelement 101 ist eben und plattenförmig erstreckt. In befestigtem Zustand (Fig. 2 bis 6) ist das Steckelement 101 koplanar in der Führungsebene P erstreckt und/oder angeordnet. Das Steckelement 101 ist orthogonal zu der Referenzebene R orientiert.

Bei der gezeigten Ausführungsform weist das Steckelement 101 einen lateralen Steckabschnitt 1011 und einen medialen Steckabschnitt 1012 auf. Die beiden Steckabschnitte 1011, 1012 können auch als laterale Platte 1011 oder laterales Paddel 1011 und dementsprechend als mediale Platte 1012 oder mediales Paddel 1012 bezeichnet werden.

Weiter weist die Befestigungseinrichtung 100 bei der gezeigten Ausführungsform ein Auflageelement 102 auf, welches orthogonal von dem Steckelement 101 in Richtung der Tasteinrichtung 200 und der Lagereinrichtung 300 abragt. Das Auflageelement 102 liegt in befestigtem Zustand des chirurgischen Instruments 1 auf der Blockoberseite 402 des Schnittblocks 400 auf (Fig. 2). Das Auflageelement ist einends mit dem Steckelement 101 und andernends mit dem besagten Schwenklager verbunden. Im Speziellen weist das Auflageelement 102 vorliegend einen lateralen Auflageabschnitt 1021 und einen medialen Auflageabschnitt 1022 auf. Der laterale Auflageabschnitt 1021 ist einends mit dem lateralen Steckabschnitt 1011 verbunden. Dementsprechend ist der mediale Auflageabschnitt 1022 einends mit dem medialen Streckabschnitt 1012 verbunden.

Die Tasteinrichtung 200 weist bei der gezeigten Ausführungsform ein um die Schwenkachse S schwenkbewegliches Strebenelement 201 auf. Das Strebenelement 201 ist entlang seiner nicht näher bezeichneten Längsrichtung zwischen einem ersten Ende 2011 und einem zweiten Ende 2012 längserstreckt. Die Referenzebene R ist auf noch näher bezeichnete Weise an einer posterior orientierten Unterseite des Strebenelements 201 angeordnet und/oder ausgebildet.

Die Schwenkachse S ist im Bereich des ersten Endes 2011 des Strebenelements 201 angeordnet und weist bei der gezeigten Ausführungsform ein Zapfenelement S1 und eine Zapfenaufnahme S2 auf. Das Zapfenelement S1 greift um die Schwenkachse S gleitbeweglich in die Zapfenaufnahme S2 ein. Vorliegend ist das Zapfenelement S1 einends an der Befestigungseinrichtung 100 angeordnet. Im Speziellen ist das Zapfenelement S1 einends und dem Befestigungselement 101 abgewandt an dem Auflageelement 102 angeordnet. Die Zapfenaufnahme S2 ist an dem ersten Ende 2011 des Strebenelements 201 angeordnet. Bei einer zeichnerisch nicht dargestellten Ausführungsform ist die Zuordnung von Zapfenaufnahme und Zapfenelement umgekehrt. Insoweit kann das Zapfenelement S1 auch an dem Strebenelement 201 und umgekehrt die Zapfenaufnahme S2 an der Befestigungseinrichtung 100 angeordnet sein.

Weiter weist die Tasteinrichtung 200 vorliegend ein Schlittenelement 202 und ein Tastelement 203 auf. Das Schlittenelement 202 ist relativ zu dem Strebenelement 201 entlang der zweiten Lagerachse L2 - die vorliegend mit der Längsachse des Strebenelements 201 zusammenfällt - translatorisch verlagerbar. Zu diesem Zweck ist das Schlittenelement 202 gleitbeweglich an dem Strebenelement 201 gelagert.

Das Strebenelement 201 weist vorliegend einen lateralen Strebenabschnitt 2013 und einen medialen Strebenabschnitt 2014 auf. Beide Strebenabschnitte 2013, 2014 sind jeweils zwischen dem ersten Ende 2011 und dem zweiten Ende 2012 längserstreckt. Die beiden Strebenabschnitte 2013, 2014 sind zueinander parallel längserstreckt. Das Schlittenelement ist entlang der zweiten Lagerachse L2 gleitbeweglich an den Strebenabschnitten 2013, 2014 geführt und weist zu diesem Zweck eine laterale Führungsaussparung 2021 und eine mediale Führungsaussparung 2022 auf. Die beiden Führungsaussparungen 2021, 2022 sind jeweils entlang der zweiten Lagerachse L2 durchgängig durch das Schlittenelement 202 erstreckt. Der laterale Strebenabschnitt 2013 ist gleitbeweglich in der lateralen Führungsaussparung 2021 aufgenommen. Der mediale Strebenabschnitt 2014 ist gleitbeweglich in der medialen Führungsaussparung 2022 aufgenommen. Das Schlittenelement 202 ist entlang der zweiten Lagerachse L2 relativ zu dem Strebenelement 201 zwischen einer ersten Endlage und einer zweiten Endlage verlagerbar. In der ersten Endlage ist das Schlittenelement 202 im Bereich des ersten Endes 2011 angeordnet. In der zweiten Endlage ist das Schlittenelement 202 im Bereich des zweiten Endes 2012 angeordnet.

Das Tastelement 203 ist orthogonal zu der Längsrichtung des Strebenelements 201 linearbeweglich an dem Schlittenelement 202 gelagert. Mit anderen Worten ist das Tastelement 203 entlang der ersten Lagerachse L1 und/oder orthogonal zu der zweiten Lagerachse L2 und/oder orthogonal zu der Referenzebene R linearbeweglich an dem Schlittenelement 202 gelagert. Das Tastelement 203 bildet einends die Referenzebene R aus. Zu diesem Zweck weist das Tastelement 203 einends einen Tastabschnitt (ohne Bezugszeichen) mit einer ersten Tastspitze 2031 und einer zweiten Tastspitze 2032 auf. Die Referenzebene R ist zwischen den beiden Tastspitzen 2031, 2032 erstreckt. Weiter weist das Tastelement 203 einen Schaftabschnitt 2033 und einen Griffabschnitt 2034 auf. Der Schaftabschnitt 2033 ist einends mit dem Tastabschnitt (ohne Bezugszeichen) und andernends mit dem Griffabschnitt 2034 verbunden. Der Schaftabschnitt 2033 ist entlang der ersten Lagerachse L1 gleitbeweglich in einer Führungsbohrung 2023 des Schlittenelements 202 geführt. Zudem ist der Schaftabschnitt 2033 vorliegend um die erste Lagerachse L1 drehbeweglich in der Führungsbohrung 2023 geführt. Der Griffabschnitt 2034 ist fest mit dem Schaftabschnitt 2033 verbunden und kann zwecks Ausrichtung der Referenzebene R beispielsweise zwischen dem Daumen und einem Finger einer Hand gegriffen werden.

Die weitere Funktion des chirurgischen Instruments 1 wird anhand Fig. 3 näher im Zusammenhang mit dem chirurgischen Instrumentensystem 10' erläutert. Dabei zeigt Fig. 3 eine intraoperative Situation, in welcher der Schnittblock 400 mittels des Referenzblocks 500 distal an dem Femur F angeordnet und ausgerichtet ist. Zu diesem Zweck ist der Referenzblock 500 seinerseits auf eine dem Fachmann grundsätzlich bekannte Weise auf einen intramedullären Stab 600 aufgeschoben. Der intramedulläre Stab 600 ist entlang einer nicht gesondert bezeichneten Längsachse des Femurs in proximaler Richtung in dessen Markraum eingebracht. Der Referenzblock 500 ist in proximaler Richtung auf ein distales Ende 601 des intramedullären Stabs 600 aufgeschoben. Dabei weist der Referenzblock 500 eine proximal orientierte Blockrückseite 501 auf. Die proximale Blockrückseite 501 ist zur Anlage an den distalen Kondylen des Femurs F eingerichtet, wobei anhand Fig. 3 lediglich die laterale distale Kondyle KL ersichtlich ist. Eine nicht näher bezeichnete Normalenrichtung der Blockrückseite 501 ist parallel zu der in Fig. 3 eingezeichneten proximodistalen Achse längserstreckt und weist in proximale Richtung.

Der Schnittblock 400 ist auf eine dem Fachmann bekannte Weise lösbar an dem Referenzblock 500 fixiert. Zu diesem Zweck weist der Referenzblock 500 eine Fixiervorrichtung 502 auf. Deren spezifischer Aufbau und Funktion sind für die vorliegende Erfindung nicht wesentlich, so dass auf weitere diesbezügliche Erörterungen verzichtet werden kann. Der Referenzblock 400 ist vorliegend derart ausgerichtet, dass die Führungsebene P des Führungsschlitzes 401 planparallel zu der Blockrückseite 501 orientiert ist. Die Führungsebene P ist insoweit mediolateral und anteroposterior eben erstreckt.

Das chirurgische Instrument 1 ist zur Kontrolle der Ausrichtung des Führungsschlitzes 401 und/oder der Führungsebene P auf die vorbeschriebene Weise lösbar an dem Schnittblock 400 befestigt. Zu diesem Zweck ist die besagte Steckverbindung zwischen dem Steckelement 101 und dem Führungsschlitz 401 ausgebildet. Die beiden Steckabschnitte 1011, 1012 sind posterior in den Führungsschlitz 401 eingesteckt. Die Schwenkachse S ist parallel zu der in Fig. 3 eingezeichneten anteroposterioren Achse ausgerichtet. Entsprechendes gilt für die erste Lagerachse L1. Die zweite Lagerachse L2 ist - in Abhängigkeit der Schwenkstellung des Strebenelements 201 um die Schwenkachse S - wenigstens überwiegend proximodistal orientiert. Die Referenzebene R ist orthogonal zu der Blockrückseite 501 orientiert.

Zur Positionierung der Referenzebene und/oder der Tastspitzen 2031, 2032 in Bezug auf die anteriore Fläche A des Femurs F kann die Tasteinrichtung 200 auf die vorbeschriebene Weise sowohl um die Schwenkachse S als auch entlang der beiden Lagerachsen L1, L2 verlagert werden. Eine translatorische Verlagerung entlang der ersten Lagerachse L1 ermöglicht eine anteroposteriore Positionierung der Referenzebene R. Eine translatorische Verlagerung entlang der zweiten Lagerachse L2 ermöglicht eine proximodistale Positionierung der Referenzebene R. Zudem kann der Tastabschnitt (ohne Bezugszeichen) mittels einer Rotation des Tastelements 203 um die erste Lagerachse L1 rotatorisch verlagert werden. Dies erlaubt eine besonders präzise Positionierung der beiden Tastspitzen 2031, 2032 in Bezug auf die anteriore Fläche A.

Eine anforderungsgerechte Ausrichtung des Schnittblocks 400 liegt insbesondere dann vor, wenn die Referenzebene R mit der anterioren Fläche A zusammenfällt. Mit anderen Worten ausgedrückt: Eine anforderungsgerechte Ausrichtung liegt insbesondere dann vor, wenn eine auf die sagittale Ebene projizierte Referenzachse (innerhalb der Referenzebene R) mit einem gleichfalls projizierten knöchernen Kurvenverlauf (innerhalb der anterioren Fläche A) zusammenfällt.

Nach erfolgter Kontrolle der Ausrichtung wird die Verbindung zwischen der Befestigungseinrichtung 100 und dem Schnittblock 400 gelöst und das chirurgische Instrument 1 wird entfernt. Hiernach kann der distale Femurschnitt angebracht werden. Hierfür wird das besagte Sägeblatt in den Führungsschlitz 401 eingeführt und die distalen Kondylen des Femurs F werden abgetrennt. Zuvor wird der Schnittblock 400 auf eine dem Fachmann bekannte Weise an dem Femur F fixiert.

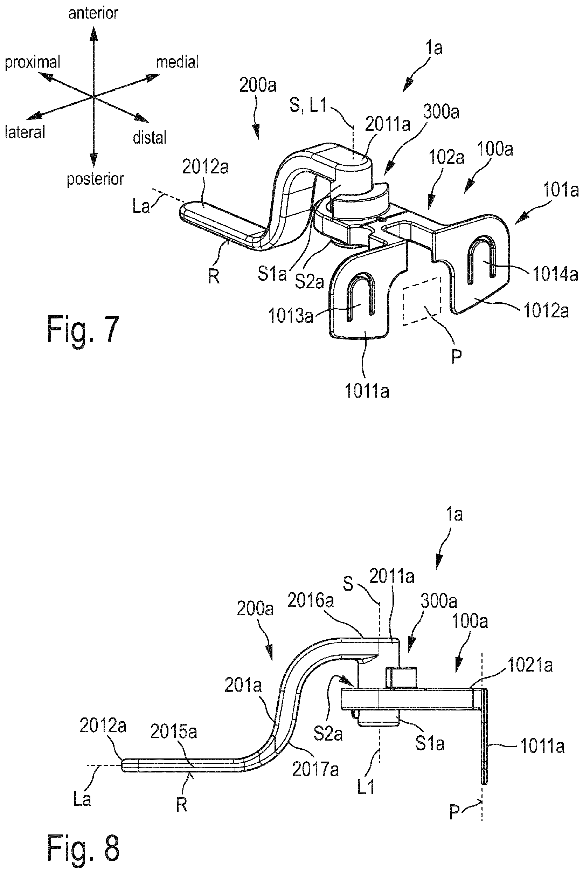

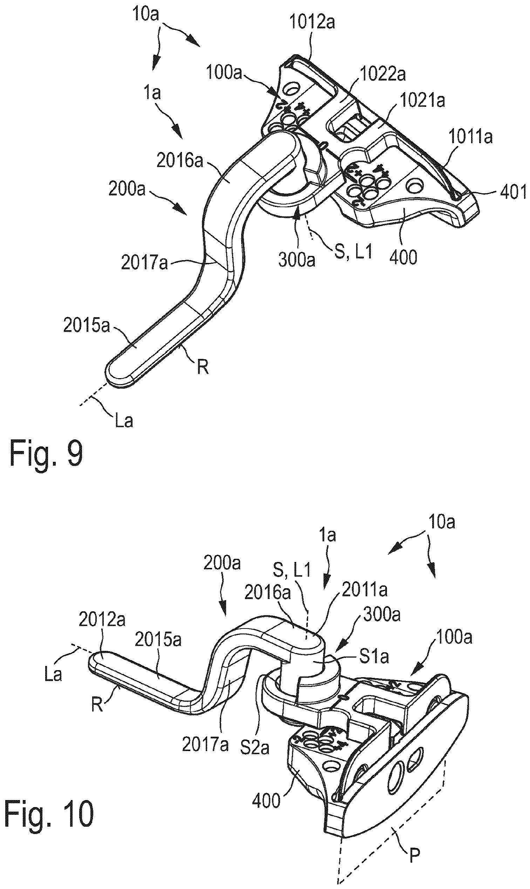

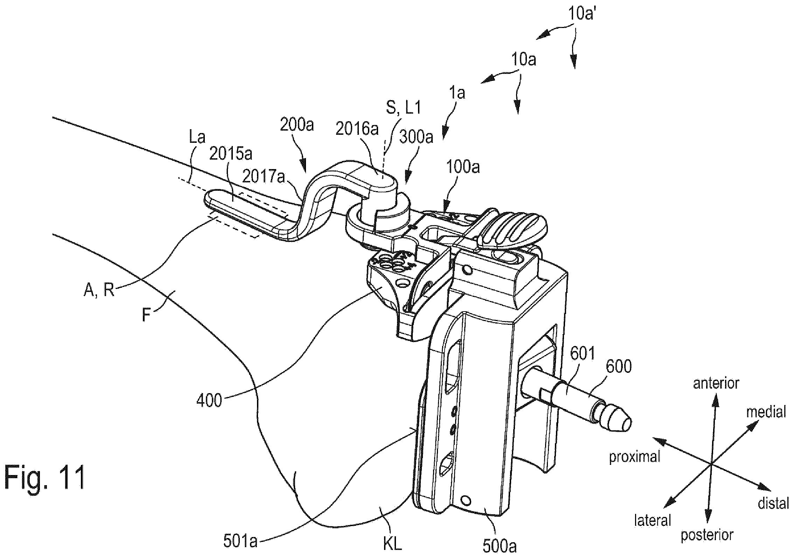

Anhand der Fig. 7 und 8 ist eine weitere Ausführungsform eines erfindungsgemäßen chirurgischen Instruments 1a gezeigt. Die Fig. 9 und 10 zeigen eine weitere Ausführungsform eines erfindungsgemäßen chirurgischen Instrumentensystems 10a, welches das chirurgische Instrument 1a nach den Fig. 7 und 8 sowie den Schnittblock 400 umfasst. Ferner zeigt Fig. 11 eine weitere Ausführungsform eines erfindungsgemäßen chirurgischen Instrumentensystems 10a', welches das chirurgische Instrumentensystem 10a nach den Fig. 9 und 10 und zudem einen Referenzblock 500a sowie den intramedullären Stab 600 umfasst. Zur Vermeidung von Wiederholungen wird nachfolgend lediglich auf wesentliche Unterschiede des chirurgischen Instruments 1a gegenüber dem chirurgischen Instrument 1 nach Fig. 1 eingegangen. Entsprechendes gilt sinngemäß für die chirurgischen Instrumentensysteme 10a, 10a'. Im Wesentlichen identische und funktionsgleiche Bauteile und/oder Abschnitte werden nicht gesondert erläutert. Stattdessen wird auf die Offenbarung zu dem chirurgischen Instrument 1 und den chirurgischen Instrumentensystemen 10, 10' ausdrücklich Bezug genommen und verwiesen.

Das chirurgische Instrument 1a unterscheidet sich in erster Linie durch eine unterschiedliche Gestaltung der Tasteinrichtung 200a und eine im Detail unterschiedliche Gestaltung der Befestigungseinrichtung 100a von dem chirurgischen Instrument 1 nach Fig. 1.

Die Tasteinrichtung 200a weist wiederum ein Strebenelement 201a auf. Das Strebenelement 201a ist entlang einer Längsachse La zwischen seinem ersten Ende 2011a und seinem zweiten Ende 2012a längserstreckt. Dabei weist das Strebenelement 201a keine durchgängig gerade, sondern stattdessen eine abgekröpfte Längserstreckung auf. Das Strebenelement 201a weist einen ersten Strebenabschnitt 2015a, einen zweiten Strebenabschnitt 2016a sowie einen dritten Strebenabschnitt 2017a auf.

Der erste Strebenabschnitt 2015a ist ausgehend von dem zweiten Ende 2012a gerade entlang der Längsachse La längserstreckt. Der erste Strebenabschnitt 2015a weist eine nicht näher bezeichnete posterior orientierte Unterseite auf, welche die Referenzebene R bildet (siehe insbesondere Fig. 8). Der zweite Strebenabschnitt 2016a ist ausgehend von dem ersten Ende 2011a parallel und anterior versetzt von dem ersten Strebenabschnitt 2015a längserstreckt. In Bezug auf die Zeichenebene der Fig. 8 ist der erste Strebenabschnitt 2015a unterhalb des zweiten Strebenabschnitts 2016a angeordnet. Der dritte Strebenabschnitt 2017a ist wenigstens überwiegend orthogonal zu der Längsachse La längserstreckt und verbindet den ersten Strebenabschnitt 2015a mit dem zweiten Strebenabschnitt 2016a.

Im Unterschied zu der Referenzebene des chirurgischen Instruments 1 nach Fig. 1 ist die Referenzebene R vorliegend nicht translatorisch entlang der Längsachse des Strebenelements 201a verlagerbar.

Ungeachtet dessen ist das Strebenelement 201a wiederum um die Schwenkachse S schwenkbeweglich an der Befestigungseinrichtung 100a gelagert. Die Schwenkachse S ist zwischen einem im Bereich des ersten Endes 2011a an dem Strebenelement 201a ausgebildeten Zapfenelement S1a und einer an der Befestigungseinrichtung 100a angeordneten Zapfenaufnahme S2a ausgebildet.

Die Lagereinrichtung 300a des chirurgische Instruments 1a weist in Übereinstimmung mit dem chirurgischen Instrument 1 nach Fig. 1 eine erste Lagerachse L1 auf. Im Unterschied fällt die erste Lagerachse L1 vorliegend mit der Schwenkachse S zusammen. Die Beweglichkeit entlang der ersten Lagerachse L1 folgt aus einer Linearverschieblichkeit zwischen dem Zapfenelement S1a und der Zapfenaufnahme S2a. Mit anderen Worten greift das Zapfenelement S1a axial gleitbeweglich in die Zapfenaufnahme S2a ein. Vorliegend ist die Linearverschieblichkeit zwischen dem Zapfenelement S1a und der Zapfenaufnahme S2a durch nicht näher bezeichnete Anschläge begrenzt. In der Anwendung des chirurgischen Instruments 1a (siehe Fig. 11) erlaubt eine gleitbewegliche translatorische Verlagerung des Zapfenelements S1a in Relation zu der Zapfenaufnahme S2a eine Einstellung der Referenzebene R in Bezug auf die in Fig. 11 gezeigte anteroposteriore Achse.

Weiter im Unterschied zu dem chirurgischen Instrument 1 nach Fig. 1 weist die Befestigungseinrichtung 100, genauer: das Steckelement 101a, vorliegend wenigstens eine Federzunge 1013a auf. Bei der gezeigten Ausführungsform sind zwei Federzungen 1013a, 1014a vorhanden, die auch als laterale Federzunge 1013a und mediale Federzunge 1014a bezeichnet werden können. Die laterale Federzunge 1013a ist an dem lateralen Steckabschnitt 1011a angeordnet. Die mediale Federzunge 1014a ist an dem medialen Steckabschnitt 1012a angeordnet. Die beiden Federzungen 1013a, 1014a sind jeweils orthogonal zu der Führungsebene P elastisch federbeweglich. In einem eingesteckten Zustand des Steckelements 101a drücken die beiden Federzungen 1013a, 1014a jeweils federkraftbelastet gegen eine nicht näher bezeichnete Innenwandung des Führungsschlitzes 401. Hierdurch wird eine verbesserte Befestigung erreicht.

Weiter in Bezug auf Fig. 11 ist erkennbar, dass der dortige Referenzblock 500a eine zu dem Referenzblock 500 des chirurgischen Instrumentensystems 10' unterschiedliche Gestaltung aufweist. Die diesbezüglichen Unterschiede sind im Hinblick auf die vorliegende Erfindung nicht von wesentlicher Bedeutung. Weitere Erläuterungen hierzu sind deshalb entbehrlich.

## Patentansprüche

1. Chirurgisches Instrumentensystem (10, 10a, 10', 10a') zur Verwendung bei einer Kniegelenkersatzoperation, aufweisend
ein chirurgisches Instrument (1, 1a) mit einer Befestigungseinrichtung (100, 100a), die zur direkten lösbaren Befestigung an einem Schnittblock (400) zur distalen Resektion eines Femurs (F) eingerichtet ist, einer Tasteinrichtung (200, 200a) mit einer posterior orientierten Referenzebene (R), die zur Anlage an einer anterioren Fläche (A) des Femurs (F) eingerichtet ist, und mit einer Lagereinrichtung (300, 300a) zur Positionierung der Referenzebene (R), wobei die Lagereinrichtung (300, 300a) wenigstens ein Schwenklager mit einer anteroposterior orientierten Schwenkachse (S) aufweist, um welche die Referenzebene (R) relativ zu der Befestigungseinrichtung (100, 100a) schwenkbeweglich verlagerbar ist, und aufweisend
einen Schnittblock (400) zur distalen Resektion des Femurs (F), wobei der Schnittblock (400) einen in einer mediolateral und anteroposterior erstreckten Führungsebene (P) angeordneten Führungsschlitz (401) zur Aufnahme und Führung eines Sägeblatts aufweist,
wobei die Referenzebene (R) der Tasteinrichtung (200, 200a) orthogonal zu der Führungsebene (P) des Führungsschlitzes (401) orientiert ist,
**dadurch gekennzeichnet, dass** die Befestigungseinrichtung (100, 100a) ein mediolateral und anteroposterior eben erstrecktes Steckelement (101, 101a) aufweist, welches form- und/oder kraftschlüssig mit dem Führungsschlitz (401) des Schnittblocks (400) zusammengesteckt ist, um die Befestigungseinrichtung (100, 100a) lösbar an dem Schnittblock (400) zu befestigen.

2. Chirurgisches Instrumentensystem (10, 10a, 10', 10a') nach Anspruch 1, **dadurch gekennzeichnet, dass** die Lagereinrichtung (300, 300a) wenigstens eine erste Linearführung aufweist, mittels derer die Referenzebene (R) relativ zu der Befestigungseinrichtung (100, 100a) translatorisch entlang einer anteroposterior orientierten ersten Lagerachse (L1) verlagerbar ist.

3. Chirurgisches Instrumentensystem (10, 10') nach Anspruch 1 oder 2, **dadurch gekennzeichnet, dass** die Lagereinrichtung (300) wenigstens eine zweite Linearführung aufweist, mittels derer die Referenzebene (R) relativ zu der Befestigungseinrichtung (100) translatorisch entlang einer wenigstens überwiegend proximodistal orientierten zweiten Lagerachse (L2) verlagerbar ist.

4. Chirurgisches Instrumentensystem (10, 10a, 10', 10a') nach einem der vorhergehenden Ansprüche, **dadurch gekennzeichnet, dass** die Tasteinrichtung (200, 200a) ein um die Schwenkachse (S) schwenkbewegliches Strebenelement (201, 201a) aufweist, welches entlang seiner Längsrichtung zwischen einem ersten Ende (2011, 2011a) und einem zweiten Ende (2012, 2012a) längserstreckt ist, wobei die Referenzebene (R) an einer posterior orientierten Unterseite des Strebenelements (201, 201a) angeordnet oder ausgebildet ist.

5. Chirurgisches Instrumentensystem (10, 10a, 10', 10a') nach Anspruch 4, **dadurch gekennzeichnet, dass** das Schwenklager eine Zapfenaufnahme (S2, S2a) und ein komplementäres Zapfenelement (S1, S1a) aufweist, welches um die Schwenkachse (S) schwenkbeweglich in der Zapfenaufnahme (S2, S2a) gelagert ist, wobei das Zapfenelement (S1, S1a) an dem ersten Ende (2011, 2011a) des Strebenelements (201, 201a) und die Zapfenaufnahme (S2, S2a) an der Befestigungseinrichtung (100, 100a) angeordnet ist oder umgekehrt.

6. Chirurgisches Instrumentensystem (10a, 10a') nach Anspruch 5, **dadurch gekennzeichnet, dass** das Zapfenelement (S1a) entlang der Schwenkachse (S), vorzugsweise begrenzt, linearbeweglich in der Zapfenaufnahme (S2a) gelagert ist.

7. Chirurgisches Instrumentensystem (10a, 10a') nach einem der Ansprüche 4 bis 6, **dadurch gekennzeichnet, dass** das Strebenelement (201a) einen ausgehend von dem zweiten Ende (2012a) gerade längserstreckten ersten Strebenabschnitt (2015a), dessen posterior orientierte Unterseite die Referenzebene (R) bildet, einen zweiten Strebenabschnitt (2016a), welcher ausgehend von dem ersten Ende (2011a) anterior parallel versetzt zu dem ersten Strebenabschnitt (2015a) längserstreckt ist, und einen dritten Strebenabschnitt (2017a), welcher wenigstens überwiegend anteroposterior längserstreckt ist und den ersten Strebenabschnitt (2015a) und den zweiten Strebenabschnitt (2016a) miteinander verbindet, aufweist.

8. Chirurgisches Instrumentensystem (10, 10') nach einem der Ansprüche 4 bis 6, **dadurch gekennzeichnet, dass** die Tasteinrichtung (200) ein Schlittenelement (202) und ein Tastelement (203) aufweist, wobei das Schlittenelement (202) relativ zu dem Strebenelement (201) in Längsrichtung des Strebenelements (201) linearbeweglich an demselben gelagert ist, wobei das Tastelement (203) relativ zu dem Schlittenelement (202) orthogonal zu der Längsrichtung des Strebenelements (201) und/oder anteroposterior linearbeweglich an dem Schlittenelement (202) gelagert ist, und wobei die Referenzebene (R) einends des Tastelements (203) angeordnet oder ausgebildet ist.

9. Chirurgisches Instrumentensystem (10, 10') nach Anspruch 8, **dadurch gekennzeichnet, dass** das Tastelement (203) relativ zu dem Schlittenelement (202) um eine anteroposterior orientierte Drehachse (D) rotierbar an dem Schlittenelement (202) gelagert ist.

10. Chirurgisches Instrumentensystem (10', 10a') nach einem der vorhergehenden Ansprüche, **dadurch gekennzeichnet, dass** ein Referenzblock (500, 500a) vorhanden ist, welcher eine proximal orientierte Blockrückseite (501, 501a) zur Anlage an distalen Kondylen des Femurs (F) aufweist, wobei der Schnittblock (400) lösbar an dem Referenzblock (500, 500a) befestigt ist, und wobei die Führungsebene (P) planparallel und die Referenzebene (R) orthogonal zu der Blockrückseite (501, 501a) orientiert sind.

## Claims

1. Surgical instrument system (10, 10a, 10', 10a') for use in a knee replacement operation, having a surgical instrument (1, 1a) with a fastening device (100, 100a) which is configured for direct releasable fastening to a cutting block (400) for the distal resection of a femur (F), a sensing device (200, 200a) with a posteriorly oriented reference plane (R) which is configured to bear against an anterior surface (A) of the femur (F), and with a bearing device (300, 300a) for positioning the reference plane (R), the bearing device (300, 300a) having at least one pivoting bearing with an anteroposteriorly oriented pivot axis (S), about which the reference plane (R) can be moved pivotably relative to the fastening device (100, 100a), and having a cutting block (400) for the distal resection of the femur (F), the cutting block (400) having a guide slot (401) which is arranged in a guide plane (P) extending mediolaterally and anteroposteriorly for receiving and guiding a saw blade,
the reference plane (R) of the sensing device (200, 200a) being oriented orthogonally with respect to the guide plane (P) of the guide slot (401),
**characterized in that** the fastening device (100, 100a) has a plug-in element (101, 101a) which is elongate in a mediolaterally and anteroposteriorly planar manner and is connected in a positively locking and/or non-positive manner to the guide slot (401) of the cutting block (400) in order to fasten the fastening device (100, 100a) releasably to the cutting block (400).

2. Surgical instrument system (10, 10a, 10', 10a') according to Claim 1, **characterized in that** the bearing device (300, 300a) has at least one first linear guide, by means of which the reference plane (R) can be moved translationally relative to the fastening device (100, 100a) along an anteroposteriorly oriented first bearing axis (L1).

3. Surgical instrument system (10, 10') according to Claim 1 or 2, **characterized in that** the bearing device (300) has at least one second linear guide, by means of which the reference plane (R) can be moved translationally relative to the fastening device (100) along an at least predominantly proximodistally oriented second bearing axis (L2).

4. Surgical instrument system (10, 10a, 10', 10a') according to one of the preceding claims, **characterized in that** the sensing device (200, 200a) has a strut element (201, 201a) which can be moved pivotably about the pivot axis (S) and is elongate along its longitudinal direction between a first end (2011, 2011a) and a second end (2012, 2012a), the reference plane (R) being arranged or configured on a posteriorly oriented lower side of the strut element (201, 201a).

5. Surgical instrument system (10, 10a, 10', 10a') according to Claim 4, **characterized in that** the pivoting bearing has a journal trunnion (S2, S2a) and a complementary journal element (S1, S1a) which is mounted in the journal trunnion (S2, S2a) such that it can be moved pivotably about the pivot axis (S), the journal element (S1, S1a) being arranged at the first end (2011, 2011a) of the strut element (201, 201a) and the journal trunnion (S2, S2a) being arranged on the fastening device (100, 100a), or vice versa.

6. Surgical instrument system (10a, 10a') according to Claim 5, **characterized in that** the journal element (S1a) is mounted in the journal trunnion (S2a) such that it can be moved linearly along the pivot axis (S), preferably to a limited extent.

7. Surgical instrument system (10a, 10a') according to one of Claims 4 to 6, **characterized in that** the strut element (201a) has a first strut portion (2015a) which is elongate in a straight line starting from the second end (2012a) and the posteriorly oriented lower side of which forms the reference plane (R), a second strut portion (2016a) which is elongate starting from the first end (2011a) in an anteriorly parallel offset manner with respect to the first strut portion (2015a), and a third strut portion (2017a) which is at least predominantly anteroposteriorly elongate and connects the first strut portion (2015a) and the second strut portion (2016a) to one another.

8. Surgical instrument system (10, 10') according to one of Claims 4 to 6, **characterized in that** the sensing device (200) has a slide element (202) and a sensing element (203), the slide element (202) being mounted on the strut element (201) such that it can be moved linearly relative to the latter in the longitudinal direction of this strut element (201), the sensing element (203) being mounted on the slide element (202) such that it can be moved linearly relative to the slide element (202) orthogonally with respect to the longitudinal direction of the strut element (201) and/or anteroposteriorly, and the reference plane (R) being arranged or configured at one end of the sensing element (203).

9. Surgical instrument system (10, 10') according to Claim 8, **characterized in that** the sensing element (203) is mounted on the slide element (202) such that it can be rotated relative to the slide element (202) about an anteroposteriorly oriented rotational axis (D).

10. Surgical instrument system (10', 10a') according to one of the preceding claims, **characterized in that** there is a reference block (500, 500a) which has a proximally oriented block rear side (501, 501a) for bearing against distal condyles of the femur (F), the cutting block (400) being fastened releasably to the reference block (500, 500a), and the guide plane (P) being oriented in a plane-parallel manner and the reference plane (R) being oriented orthogonally with respect to the block rear side (501, 501a).

## Revendications

1. Système d'instrument chirurgical (10, 10a, 10', 10a') destiné à être utilisé dans une opération d'arthroplastie du genou, présentant
un instrument chirurgical (1, 1a) présentant un dispositif de fixation (100, 100a), qui est conçu pour une fixation directe amovible à un guide de coupe (400) pour la résection distale d'un fémur (F), un dispositif palpeur (200, 200a) présentant un plan de référence (R) orienté de manière postérieure, qui est conçu pour venir en butée contre une surface antérieure (A) du fémur (F) et présentant un dispositif support (300, 300a) pour le positionnement du plan de référence (R), le dispositif support (300, 300a) présentant au moins un palier pivotant pourvu d'un axe de pivotement (S) orienté de manière antéropostérieure autour duquel le plan de référence (R) peut se déplacer de manière pivotante par rapport au dispositif de fixation (100, 100a) et présentant
un guide de coupe (400) pour la résection distale du fémur (F), le guide de coupe (400) présentant une fente de guidage (401) disposée dans un plan de guidage (P) s'étendant de manière médiolatérale et antéropostérieure pour la réception et le guidage d'une lame de scie,
le plan de référence (R) du dispositif palpeur (200, 200a) étant orienté orthogonalement par rapport au plan de guidage (P) de la fente de guidage (401), **caractérisé en ce que** le dispositif de fixation (100, 100a) présente un élément enfichage (101, 101a) s'étendant de manière médiolatéralement et antéropostérieurement plane, qui est enfiché par complémentarité de forme et/ou à force dans la fente de guidage (401) du guide de coupe (400), afin de fixer le dispositif de fixation (100, 100a) de manière amovible au guide de coupe (400).

2. Système d'instrument chirurgical (10, 10a, 10', 10a') selon la revendication 1, **caractérisé en ce que** le dispositif support (300, 300a) présente au moins un premier guidage linéaire au moyen duquel le plan de référence (R) peut être déplacé en translation par rapport au dispositif de fixation (100, 100a) le long d'un premier axe support (L1) orienté de manière antéropostérieure.

3. Système d'instrument chirurgical (10, 10') selon la revendication 1 ou 2, **caractérisé en ce que** le dispositif support (300) présente au moins un deuxième guidage linéaire au moyen duquel le plan de référence (R) peut être déplacé en translation par rapport au dispositif de fixation (100) le long d'un deuxième axe support (L2) orienté au moins principalement de manière proximodistale.

4. Système d'instrument chirurgical (10, 10a, 10', 10a') selon l'une des revendications précédentes, **caractérisé en ce que** le dispositif palpeur (200, 200a) présente un élément de montant (201, 201a) pouvant se déplacer de manière pivotante autour de l'axe de pivotement (S), lequel élément est orienté longitudinalement le long de sa direction longitudinale entre une première extrémité (2011, 2011a) et une deuxième extrémité (2012, 2012a), le plan de référence (R) étant disposé ou réalisé au niveau d'une face inférieure orientée de manière postérieure de l'élément de montant (201, 201a).

5. Système d'instrument chirurgical (10, 10a, 10', 10a') selon la revendication 4, **caractérisé en ce que** le palier pivotant présente un logement de tenon (S2, S2a) et un élément de tenon (S1, S1a) complémentaire, qui est monté de manière à pouvoir se déplacer de manière pivotante autour de l'axe de pivotement (S) dans le logement de tenon (S2, S2a), l'élément de tenon (S1, S1a) étant disposé au niveau de la première extrémité (2011, 2011a) de l'élément de montant (201, 201a) et le logement de tenon (S2, S2a) étant disposé au niveau du dispositif de fixation (100, 100a) ou inversement.

6. Système d'instrument chirurgical (10a, 10a') selon la revendication 5, **caractérisé en ce que** l'élément de tenon (S1a) est monté de manière à pouvoir se déplacer linéairement, de préférence de manière limitée, le long de l'axe de pivotement (S) dans le logement de tenon (S2a).

7. Système d'instrument chirurgical (10a, 10a') selon l'une des revendications 4 à 6, **caractérisé en ce que** l'élément de montant (201a) présente une première section de montant (2015a) orientée de manière longitudinalement droite partant de la deuxième extrémité (2012a), dont la face inférieure orientée de manière postérieure forme le plan de référence (R), une deuxième section de montant (2016a), qui s'étend longitudinalement, partant de la première extrémité (2011a), de manière antérieurement et parallèlement décalée par rapport à la première section de montant (2015a) et une troisième section de montant (2017a), qui s'étend longitudinalement au moins principalement de manière antéropostérieure et qui relie l'une à l'autre la première section de montant (2015a) et la deuxième section de montant (2016a).

8. Système d'instrument chirurgical (10, 10') selon l'une des revendications 4 à 6, **caractérisé en ce que** le dispositif palpeur (200) présente un élément coulisseau (202) et un élément palpeur (203), l'élément coulisseau (202) étant monté sur l'élément de montant (201) de manière à pouvoir se déplacer linéairement par rapport à celui-ci dans la direction longitudinale de l'élément de montant (201), l'élément palpeur (203) étant monté sur l'élément coulisseau (202) de manière à pouvoir se déplacer linéairement par rapport à l'élément coulisseau (202), orthogonalement par rapport à la direction longitudinale de l'élément de montant (201) et/ou de manière antéropostérieure, et le plan de référence (R) étant disposé ou réalisé à une extrémité de l'élément palpeur (203).

9. Système d'instrument chirurgical (10, 10') selon la revendication 8, **caractérisé en ce que** l'élément palpeur (203) est monté sur l'élément coulisseau (202) de manière rotative par rapport à l'élément coulisseau (202) autour d'un axe de rotation (D) orienté de manière antéropostérieure.

10. Système d'instrument chirurgical (10', 10a') selon l'une des revendications précédentes, **caractérisé en ce qu'**un guide de référence (500, 500a) est présent, qui présente une face arrière (501, 501a) de guide, orientée de manière proximale, destinée à venir en butée contre les condyles distaux du fémur (F), le guide de coupe (400) étant fixé de manière amovible au guide de référence (500, 500a) et le plan de guidage (P) étant orienté de manière plan-parallèle et le plan de référence (R) étant orienté orthogonalement par rapport à la face arrière (501, 501a) de guide.
